# EUROPEAN PATENT SPECIFICATION

(11) **EP 4 366 654 B1**
(45) Date of publication and mention of the grant of the patent: **22.01.2025**
(21) Application number: 22744174.8
(22) Date of filing: 05.07.2022
(51) Int. Cl.: A61C 7/08, A61F 5/56

(54) **AN ORTHODONTIC ALIGNER**
KIEFERORTHOPÄDISCHER ALIGNER
ALIGNEUR ORTHODONTIQUE

(30) Priority: 07.07.2021 EP 21184167
(43) Date of publication of application: 15.05.2024
(73) Proprietor: Koninklijke Philips N.V., 5656 AG Eindhoven (NL)
(72) Inventor: ELLIOTT-BOWMAN, Bernadette, 5656 AG Eindhoven (NL); TASSIGNON, Tom, 5656 AG Eindhoven (NL)
(74) Representative: Philips Intellectual Property & Standards
(86) International application number: PCT/EP2022/068502
(87) International publication number: WO 2023/280801

(56) References cited:
- WO-A1-2017/218947
- US-A1- 2013 140 289
- US-A1- 2019 099 129

## Description

### FIELD OF THE INVENTION

This invention relates to orthodontic aligners.

### BACKGROUND OF THE INVENTION

Like traditional braces, orthodontic aligners are designed to move the teeth a little at a time. The aligners are designed based on the particular oral geometry of a user and wearing the aligner puts gentle pressure on the teeth, slightly repositioning them over time.

Patient compliance can significantly impact the effectiveness of orthodontic alignment treatments, particularly in the case of removable aligners. Healthcare professionals are currently limited in their ability to monitor treatment compliance and progression, relying on techniques that do not allow problems to be identified in a timely manner, provide limited information and can easily be spoofed by the patient.

It would be desirable to incorporate sensing functionality to enable monitoring of compliance and/or monitoring the tooth movement.

However, orthodontic aligners are compact so that they provide the least discomfort when worn, and they are disposable items. These factors make it challenging to incorporate meaningful smart features such as sensing functionality.

WO 2017/218947 discloses an aligner which incorporates sensing functionality to detect wearing of the aligner and hence compliance. A sensor is provided at the aligner and it communicates using near field communication with an external monitoring unit.

### SUMMARY OF THE INVENTION

The invention is defined by the claims.

According to examples in accordance with an aspect of the invention, there is provided an orthodontic aligner, comprising:
a support structure for placing in the mouth of a user, wherein the support structure has a shape which fits to the teeth of the user and has a deformable region; and
at least one passive RFID tag mounted to the support structure, comprising first and second portions coupled to the deformable region, wherein the first and second portions each comprise a respective antenna portion, which together define the antenna for the RFID tag,
wherein a relative position between the first and second portions is adjustable by deformation of the deformable region between at least a first relative position when the orthodontic aligner is worn by the user and a second relative position when the orthodontic aligner is not being worn,
wherein a response of the RFID tag to an external interrogation signal is dependent on the relative position between the first and second portions.

The invention in this way provides a smart aligner, which can be interrogated externally to determine if it is worn. In this way, aligner compliance (and optionally also tracking of the correct fit) is enabled. The deformation of the deformable region caused by the presence of the teeth results in a detectable change in the RFID response, in particular by changing the effective antenna structure of the RFID tag. The two portions of the RFID tag in particular define different portions e.g. halves of an RFID antenna. The aligner is entirely passive and hence low cost. For example the RFID tag is passive and simply responds to an interrogation signal without requiring a power supply; the energy for operation is harvested from the interrogation signal.

When the aligner is not being worn, the first and second portions remain separate, i.e. they do not form a complete RFID tag. However, when the aligner is worn correctly, the deformable region changes shape slightly such that the portions come into contact forming a working, passive RFID tag.

Information about how the aligner is being worn can then be detected based on the external interrogation, for example by sending an RF pulse to the aligner, and analyzing the backscattered signals for the signature of the RFID antenna. The system thereby allows wearing of the aligner to be monitored remotely and in real time, and the information gathered can be sent to the user (patient) or their dentist to improve orthodontic outcomes.

In this way, the system allows dental professionals and/or patients to gather new and advantageous information about how an orthodontic aligner treatment plan is progressing using cheap and readily-available technology.

The RFID technology used to implement the system is increasingly small, cheap and easy to manufacture.

The aligner may comprise a plurality of RFID tags, each comprising first and second portions coupled to the deformable region.

The correct fitting of the aligner can thereby be determined by verifying that the deformation has taken place at all required positions of the support structure.

There may even be a plurality of RFID tags associated with one or more individual teeth to be monitored. Thus, tooth movement of a tooth can be monitored based on the RFID tags out of a set that a currently active. There may be multiple teeth, each with a set of RFID tags to enable monitoring of tooth position and movement.

The relative position between the first and second portions is for example adjustable between a first state in which the RFID tag is non-functional and will not respond to an interrogation signal and a second state in which RFID tag is functional and will respond to an interrogation signal. Thus, the presence of a response is indicative that the aligner is worn (at the location of the RFID tag).

The RFID tag is for example functional when the first and second portions are brought into physical electrical contact with each other. The portions may have dedicated contacts with engage with each other.

The support structure for example comprises a guard (i.e. a shell aligner) that fits over the teeth of one jaw. There may then be two such guards.

The deformable region may include a surface which makes contact with occlusal tooth surfaces, and at least on RFID tag comprises upper and lower portions which move together when the aligner is worn.

Thus, when the guard is fitted, the biting surfaces of the teeth push down on the deformable region so that the RFID portions move together.

The deformable region may alternatively or additionally include a surface which faces the labial tooth surfaces, and at least one RFID tag comprises inner and outer portions which move together when the aligner is worn.

Thus, when the guard is fitted, the front surface of the front teeth push outwardly on the deformable region so that the RFID portions move together.

The invention also provides an aligner system, comprising:
the aligner as defined above;
an RFID interrogator unit for interrogating the one or more RFID tags and receiving a reflected response; and
a controller for determining whether the aligner is worn from the reflected response.

The RFID interrogator and controller of the system are for example remote from the parts of the aligner which are worn in the mouth, for example implemented as a smartphone or other wireless electronic device.

The controller is for example also adapted to determine a level of fit of the aligner to the user from the received reflected responses.

By gathering information about how the aligner is fitting, orthodontic treatment plans can be adjusted dynamically to optimize the treatment and respond to patients' needs, improving the effectiveness of the alignment process by identifying and implementing interventions earlier.

The controller may be adapted to determine tooth position information from the received reflected response. By gathering information about how the teeth have moved over time, from position information at different times, orthodontic treatment plans can again be adjusted dynamically to optimize the treatment.

The disclosure also provides a method (not claimed) of detecting if an orthodontic aligner is currently being worn, comprising:
generating a control signal for controlling an RFID interrogator unit (20) to generate an RFID interrogation signal for delivery to one or more passive RFID tags forming part of the aligner; and
receiving a signal based on the reflected response from the passive RFID tag or tags and analyzing the reflected response signal, thereby to detect antenna characteristics of the RFID tag which depend on a relative position between first and second portions of the RFID tag, and thereby determining if the orthodontic aligner is being worn.

The full method thus comprises:
generating an RFID interrogation signal;
using an RFID interrogator unit to deliver the RFID interrogation signal to one or more passive RFID tags forming part of an the aligner; and
analyzing a reflected response from the RFID tag or tags, thereby to detect antenna characteristics of the RFID tag which depend on a relative position between first and second portions of the RFID tag, and thereby determining if the orthodontic aligner is being worn.

The disclosure also provides a computer program (not claimed) at comprising computer program code which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows an orthodontic aligner;
Figure 2 shows a first example of possible RFID tag design fitted to the support structure of an aligner;
Figure 3 shows a second example of possible RFID tag design fitted to the support structure of an aligner;
Figure 4A shows how wearing an aligner can be detected;
Figure 4B shows how tooth movement may be detected; and
Figure 5 is a schematic overview of the overall tooth aligner and monitoring system.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an orthodontic aligner which includes a passive RFID tag comprising first and second portions coupled to a deformable region of the aligner, each portion comprising a respective antenna portion which together define the antenna for the RFID tag. The relative position between the first and second portions is changed by deformation of the deformable region when the aligner is worn by the user, so that a response of the RFID tag (in particular the ability of the tag to respond) to an external interrogation is dependent on whether or not the aligner is being worn.

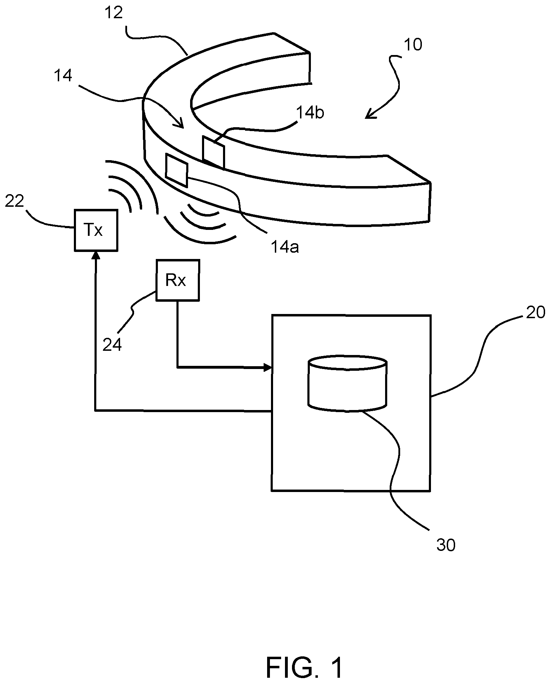

Figure 1 shows an orthodontic aligner 10, comprising a support structure 12 for placing in the mouth of a user. The support structure has a shape which fits to the teeth of the user. At least one passive RFID tag 14 is mounted to the support structure, comprising first and second portions 14a, 14b. These portions are coupled to a deformable region of the support structure so that they may move relative to each other to accompany deformation of the deformable region.

The first and second portions 14a, 14b each comprise a respective antenna portion, and these antenna portions together define the antenna for the RFID tag 14.

A relative position between the first and second portions 14a, 14b is adjustable by deformation of the deformable region between at least a first relative position when the orthodontic aligner is worn by the user and a second relative position when the orthodontic aligner is not being worn.

An RFID interrogator unit 20 uses a transmitter 22 to generate an external interrogation signal and receives a return signal via a receiver 24. The received signal is dependent on the relative position between the first and second portions 14a, 14b. In particular, the relative position between the first and second portions is adjustable between a first state in which the RFID tag is non-functional because the antenna is not connected so that the RFID tag will not respond to an interrogation signal, and a second state in which RFID tag is functional and will respond to an interrogation signal.

The invention makes use of RFID technology. Advances in RFID technologies mean that printed RFID tags can be used to passively indicate touch points. When the antenna portions are separate, they do not create backscatter from an incident signal, but when brought into electrical contact they reflect a distinctive signature and hence can be used to detect relative motion between the two portions.

Passive RFID tags can be embedded in harsh environments. They can be cheap and flexible by using printed electronics techniques. For example, RFID tags can be manufactured using inkjet printing and screen printing techniques.

The technology needed for the interrogator unit 20 is also widespread. 5G communications technology is being rolled out to consumers globally, which is expected to increase the number of devices with ultra-high frequency (UHF) antennae in the home (such as smartphones and routers). This advance will increase the number of devices in the home that are capable of interfacing with passive RFID technologies.

The deformable region of the support structure may comprise a soft layered structure whereby tooth-induced deformation may cause two layers to touch. A soft flap or hinged structure may instead be used, whereby tooth-induced deformation may cause the flap to make contact with another surface.

The aligner may have a plurality of RFID tags, each comprising first and second portions coupled to the deformable region of the support structure 12. Contact may be monitored at a set of points around the shape of the aligner. However, in addition, contact may be monitored using a plurality of RFID tags associated with one or more individual teeth to be monitored. Thus, different tooth positions may trigger different contacts to be made and broken and thereby activate and deactivate different RFID tags.

The RFID tag portions may for example be around 1mm apart in the non-contact situation. There may be a single RFID chip and two antenna portions, but there could also be separate circuit portions in two separate chips, which only connect when the two portions connect. Thus, a first RFID tag portion may comprise a first chip and a first antenna portion and the second RFID tag portion then comprises a second chop and a second antenna portion.

The two antenna portions may be two portions of a dipole antenna.

The monitoring may therefore be used to monitor aligner wear compliance (i.e. monitor when the aligner is worn and when it is not), aligner fit (i.e. monitor that contact is made at the correct locations representing a correct placement of the aligner) and alignment progression (i.e., tooth movement).

Each RFID tag responds to an external interrogation by means of a unique code so that the responding RFID tags are identified. Multiple responding RFID tags can thus be identified.

The number, location and configuration of the RFID tags depend on the desired detection capability of the aligner. For example, a single RFID tag may be sufficient to indicate compliance (when the aligner is in the mouth, the single RFID tag is activated). An array of two or more RFID tags may be used to indicate aligner fit and/or tooth movement. The correct aligner fitting may for example be separately detected at different areas, for example at the front of the mouth and at the rear.

In order to design the placement of the RFID tags, a design algorithm may be used to design the geometry and behavior of the RFID tags on the aligner. The design algorithm for example takes as inputs:
(i) Patient data describing the current, past or future state of the patient's teeth, for example:
   Dental impressions;
   3D models;
   Diagnoses or other information provided by a dental professional;
   X-ray data;
   Previous aligner designs; and
   Any other relevant information about the Patient's dental or general health.
(ii) Treatment data, for example:
   The Patient's orthodontic alignment goals such as the anticipated final (orthodontically corrected) positions of teeth and anticipated movement of teeth during the treatment process;
   Models for how the Patient's teeth will move due to wearing the aligner; and
   Relevant information pertaining to circumstances under which the treatment is administered.
(iii) Data collected by previously used aligners used before the current one in the course of a treatment program.

The design algorithm for example produces as outputs:
(i) The configuration of the support structure, for example:
   The geometry of the support structure;
   The fit of the support structure over the patient's teeth;
   The material and geometric properties of the aligner which determine how it deforms upon contact with teeth;
   The size, shape and appearance of the aligner in the patient's mouth; and
   The location of the deformable regions (e.g. flaps) with respect to the overall shape of the support structure.
(ii) The configuration of the RFID tags on the aligner, for example:
   The number of RFID tags;
   The positions and rotational orientations of the RFID tags relative to expected or known locations of teeth and the support structure.

The design algorithm then ensures that the locations and deformation properties of the support structure will be such that the extent and characteristics of deformation will actuate the RFID tags allowing meaningful measurements to be extracted.

In the design of the aligner, weights may be assigned to different aspects to prioritize critical features. Assigned weights may be on the basis of known dental best practice or customized to the patient's orthodontic goals and preferences.

For example, correct orthodontic alignment may be weighed with a maximum multiplier of 5. Effective placement of the RFID tags to record specific aligner usage properties may be weighed with a multiplier of 4. Minimal visibility of the RFID tags or support structure to onlookers may be weighed with a multiplier of 2, etc.

In use, with the designed aligner placed in the mouth over the teeth, correct contact with the teeth causes the support structure to deform, altering the passive response of the associated RFID tags. The characteristics of this deformation are determined by the relative positions of the patient's teeth and the aligner, the geometrical and material properties of the aligner (with constraint that the deformation does not impact on the rigidity required to provide orthodontic correction).

The RFID interrogator unit detects and analyzes the state of the aligner, and thereby deduces compliance and fit quality. The RFID interrogation unit may be embodied in a third-party device such as a smartphone.

In order to interpret the RFID signals that are received, the RFID interrogator unit 20 comprises a database 30 which stores information about the expected RFID signals depending on exactly how the aligner is placed relative to the patient's teeth. The database 30 for example comprises a look-up table describing how the RFID tag configurations map to different received signals. Simulations may be provided of how the RFID signals will look in various configurations.

The received RFID signals at the interrogator unit are analyzed using the database to deduce whether the aligner is in proper alignment with the patient's teeth. For example, each RFID tag will have an ID and a location (e.g. top aligner tray, rear right) is associated with each ID. The RFID tag signal (e.g. open signal X or closed signal Y) is used to determine an aligner condition (signal X: loose fit, signal Y: correct fit).

The analysis then deduces whether and in what localities the aligner fits, or where the fit has changed. A map is for example stored between the RFID tag information and the aligner geometry. Detecting which RFID tags are in the closed state can therefore be translated into knowledge of which localities of the aligner are properly aligned with the patient's teeth.

The analysis of the responses to the tags may use machine learning techniques. It may generate an alert to communicate relevant information to the patient or a relevant healthcare professional. The alert may comprise notifications (via a smartphone, wearable, the aligner itself or another device) provided to the patient. An alert may comprise a prompt to wear the aligner when it is found to not be in the mouth, or a prompt when the aligner is in the mouth but not fitted correctly giving instructions on where the fit should be adjusted.

Historical information may be shared with a healthcare professional detailing how long an aligner has been worn by a patient, and how the patient's teeth have moved in response.

The aligner patient and their orthodontic health professional may be informed of dental issues and progress in real time.

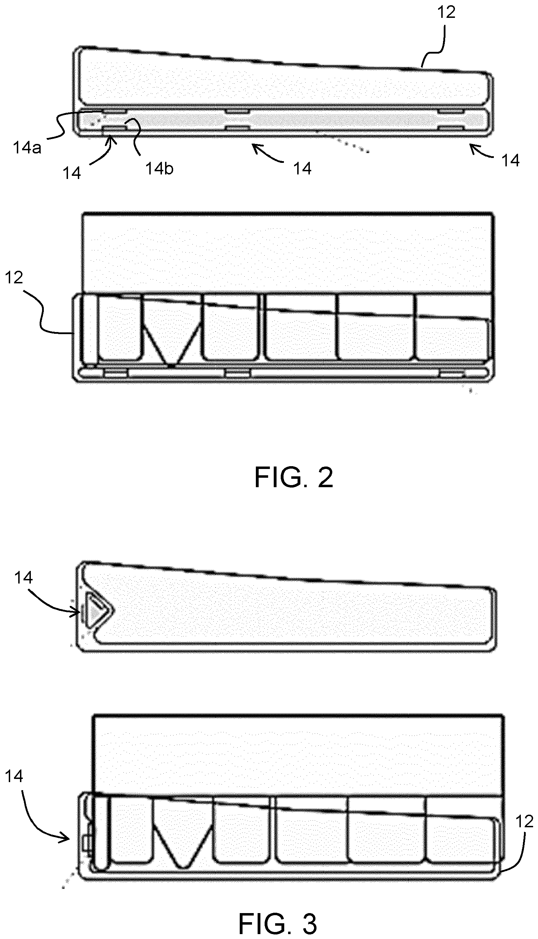

Figure 2 shows an example of possible RFID tag design fitted to the support structure 12 of an aligner.

The top image shows the aligner before fitting to the teeth. Three RFID tags 14 are shown, where there is no contact between the two portions 14a, 14b of each RFID tag. The deformable region of the support structure includes a surface which makes contact with occlusal (biting) tooth surfaces. The RFID tags comprise upper and lower portions which move together when the aligner is worn.

The aligner in the worn state is shown in the bottom image. This is a side view of the mouth, showing (from left to right) two incisors (one in side view), the canine, the two premolars and two molars.

Figure 3 shows another example of possible RFID tag design fitted to the support structure 12 of an aligner.

The top image again shows the aligner before fitting to the teeth. One RFID tags 14 is shown, and there is no contact between the two portions of the RFID tag. The deformable region includes a surface which faces the labial tooth surfaces (in particular of a front incisor), and the RFID tag comprises inner and outer portions which move together when the aligner is worn.

The aligner in the worn state is shown in the bottom image. This is again a side view of the mouth.

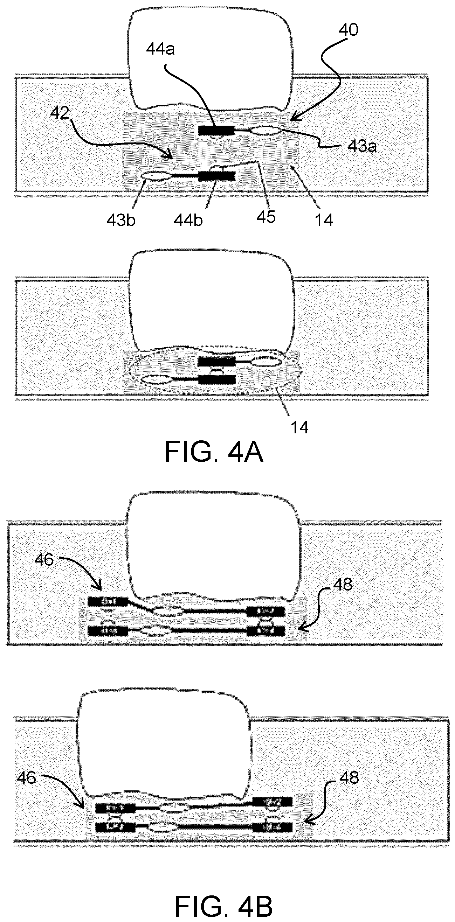

Figure 4A shows how wearing the aligner can be detected. One RFID tag 14 is shown beneath the occlusal surface of a tooth. It is split into a top RFID tag portion 40 (with a first RFID circuit 44a and a first antenna portion 43a) and a bottom RFID tag portion 42 (with a second RFID circuit 44b and a second antenna portion 43b). The top image shows a first tooth position in which there is no contact between the RFID tag portions, and the bottom image shows contact. The contact is for example made between contact pads of the RFID circuits.

Instead of each RFID tag having independent antenna halves, there may an antenna portion associated with each RFID tag chip, but then a shared remaining antenna which spans the whole aligner.

Figure 4B shows how tooth movement may be detected. Two RFID tags 46, 48 are shown beneath the occlusal surface of a tooth.

The tag 46 has two circuit portions and two antenna portions and the tag 48 has two circuit portions and two antenna portions. However, the antenna portions are shared.

The top image shows a first tooth position in which one of the RFID tags is activated, and the bottom image shows how tooth movement means a second tooth position caused the other of the RFID tags to be activated.

The same antenna is used by the two RFID tags, but only one connected pair of circuit portions is connected, and these determine the response that is made to the interrogation signal.

The monitoring of tooth movement could be enabled by understanding which combinations of RFID chips in an array or string are in electrical contact at a given time, and how this evolves over the course of treatment. In an embodiment with this functionality, it is likely that each tooth (or a subset of teeth) would be associated with two half-antennae as shown in Figure 4B, each in electrical contact with as many RFID chips as is required to achieve the required spatial resolution, this with the antenna sharing as explained above. The positioning of the RFID tags would be designed on a case-by-case basis, depending on specific parameters of interest to e.g. a health professional. The specifics would be designed to take account of, for example, the geometry of a patient's mouth, and knowledge about the material properties of the deformable structure, such that the geometry of the deformable region may be designed to produce desired functionality. It is not envisioned that any additional sensors would be included on the device for the purpose of tooth movement monitoring.

In the preferred implementation, the two halves of an RFID antenna are not in electrical contact when the RFID tag is to be non-responsive, and the deformation of the support structure is such that two halves of the antenna are brought together, either directly or by means of their associated RFID circuit portions (as in Figures 4A and 4B) to form a functional whole when the RFID tag is to be responsive to an interrogation signal generated by the transmitter of the interrogator unit.

The interrogation signals may be generated at regular intervals or sporadically, and a detection system may additionally detect when the aligner is close to the patient's face through gesture recognition technologies that are known in the art. This can be used to save power. The signals are preferably of a form which prevent the patient from simulating compliance.

Where an array of RFID tags is used, a sequence of interrogation signals may be used with differing properties in order to apply unique tests to each RFID tag.

When an RFID tag is responsive, it will modulate an interrogation signal in a detectable way, typically encoding information on the transmitted signal with information identifying that particular RFID tag.

In the preferred implementation, a tag is either responsive or non-responsive. However, the deformation of the support structure may activate an additional component.

The data processing of the RFID tag signals may take place on a second device (such as the patient's smartphone) or in a cloud location.

The output (feedback information) may indicate whether the aligner is worn correctly or at all, whether the patient's teeth as moving as required over the course of treatment, historical details on how, when and for how long the aligner is being worn. The feedback may be by means of smartphone notifications to the patient, or by haptic feedback through the aligner to the patient. There may be communication to the healthcare professional under whose guidance the patient is undergoing treatment.

The timing and frequency of feedback may be optimized, for example a patient may be notified immediately on detection of incorrect aligner wear, in order to remedy the problem in real time. A dentist may be notified of the patient's compliance with the orthodontic treatment at intervals of days or weeks, such that sufficient data has been gathered from the aligner to provide a useful temporal analysis of patient behavior.

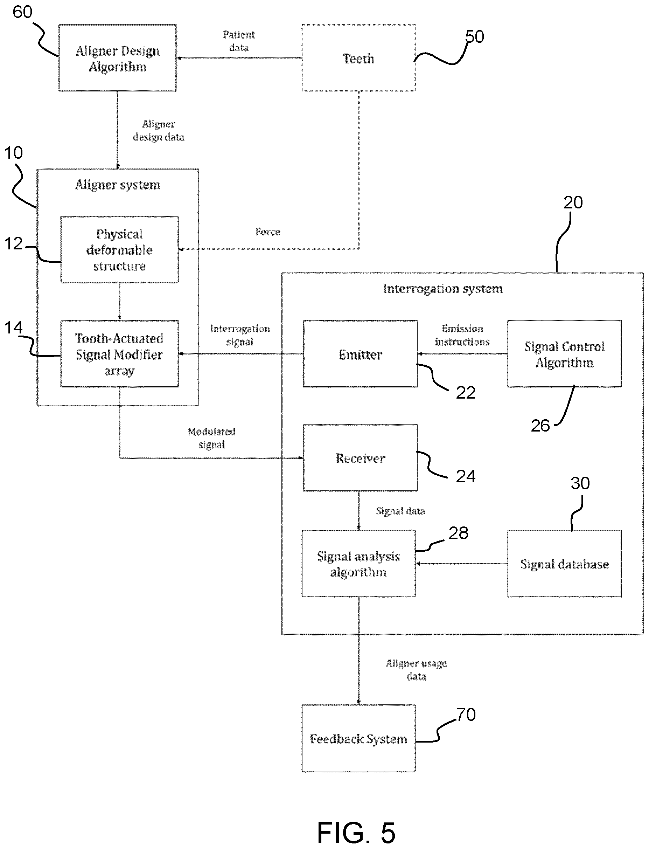

Figure 5 is a schematic overview of the overall system.

The aligner 10 comprises the support structure 12, functioning as a physically deformable structure, and the array of RFID tags, functioning as a tooth-actuated signal modifier array.

The patient's teeth are represented by block 50, and patient data relating to the teeth is provided to the aligner design algorithm 60.

The interrogator unit 20 comprises the RF transmitter (emitter) 22, the RF receiver 24 and the database 30. The transmitter is controlled by a signal control algorithm 26 and the received signals are interpreted by a signal analysis algorithm 28. This analysis results in the feedback from the feedback system 70, providing aligner usage data.

## Claims

1. An orthodontic aligner (10), comprising:
a support structure (12) for placing in the mouth of a user, wherein the support structure has a shape which fits to the teeth of the user and has a deformable region; and
at least one passive RFID tag (14) mounted to the support structure,
**characterised in that** the passive RFID tag comprises first and second portions (14a, 14b) coupled to the deformable region, wherein the first and second portions each comprise a respective antenna portion, which together define the antenna for the RFID tag,
wherein a relative position between the first and second portions is adjustable by deformation of the deformable region between at least a first relative position when the orthodontic aligner is worn by the user and a second relative position when the orthodontic aligner is not being worn,
wherein a response of the RFID tag to an external interrogation signal is dependent on the relative position between the first and second portions.

2. The aligner of claim 1, comprising a plurality of RFID tags (14), each comprising first and second portions coupled to the deformable region.

3. The aligner of claim 2, comprising a plurality of RFID tags (14) associated with one or more individual teeth to be monitored.

4. The aligner of any one of claims 1 to 3, wherein the relative position between the first and second portions is adjustable between a first state in which the RFID tag is non-functional and will not respond to an interrogation signal and a second state in which RFID tag is functional and will respond to an interrogation signal.

5. The aligner of any one of claims 1 to 4, wherein the support structure (12) comprises a guard that fits over the teeth of one jaw.

6. The aligner of claim 5, wherein the deformable region includes a surface which makes contact with occlusal tooth surfaces, and at least on RFID tag comprises upper and lower portions (14a, 14b) which move together when the aligner is worn.

7. The aligner of claim 5 or 6, wherein the deformable region includes a surface which faces the labial tooth surfaces, and at least on RFID tag comprises inner and outer portions which move together when the aligner is worn.

8. An aligner system, comprising:
the aligner (10) of any one of claims 1 to 7;
an RFID interrogator unit (20) for interrogating the one or more RFID tags and receiving a reflected response; and
a controller (28) for determining whether the aligner is worn from the reflected response.

9. The system of claim 8, wherein the controller (28) is adapted to determine a level of fit of the aligner to the user from the received reflected response.

10. The system of claim 8 or 9, wherein the controller (28) is adapted to determine tooth position information from the received reflected response.

## Patentansprüche

1. Kieferorthopädischer Aligner (10), umfassend:
eine Stützstruktur (12) zum Platzieren im Mund eines Benutzers, wobei die Stützstruktur eine Form aufweist, die zu den Zähnen des Benutzers passt, und einen verformbaren Bereich aufweist; und
mindestens einen passiven RFID-Tag (14), der an der Stützstruktur montiert ist, **dadurch gekennzeichnet, dass** der passive RFID-Tag einen ersten und einen zweiten Abschnitt (14a, 14b) umfasst, die mit dem verformbaren Bereich gekoppelt sind, wobei der erste und der zweite Abschnitt jeweils einen entsprechenden Antennenabschnitt umfassen, die zusammen die Antenne für den RFID-Tag definieren,
wobei eine relative Position zwischen dem ersten und dem zweiten Abschnitt durch Verformung des verformbaren Bereichs zwischen mindestens einer ersten relativen Position, wenn der kieferorthopädische Aligner vom Benutzer getragen wird, und einer zweiten relativen Position, wenn der kieferorthopädische Aligner nicht getragen wird, einstellbar ist,
wobei eine Reaktion des RFID-Tags auf ein externes Abfragesignal von der relativen Position zwischen dem ersten und dem zweitem Abschnitt abhängt.

2. Aligner nach Anspruch 1, umfassend eine Vielzahl von RFID-Tags (14), die jeweils einen ersten und einen zweiten Abschnitt umfassen, die mit dem verformbaren Bereich gekoppelt sind.

3. Aligner nach Anspruch 2, umfassend eine Vielzahl von RFID-Tags (14), die mit einem oder mehreren einzelnen zu überwachenden Zähnen verbunden sind.

4. Aligner nach einem der Ansprüche 1 bis 3, wobei die relative Position zwischen dem ersten und dem zweiten Abschnitt zwischen einem ersten Zustand, in dem der RFID-Tag nicht funktionsfähig ist und auf ein Abfragesignal nicht reagiert, und einem zweiten Zustand, in dem der RFID-Tag funktionsfähig ist und auf ein Abfragesignal reagiert, einstellbar ist.

5. Aligner nach einem der Ansprüche 1 bis 4, wobei die Stützstruktur (12) einen Schutz umfasst, der über die Zähne eines Kiefers passt.

6. Aligner nach Anspruch 5, wobei der verformbare Bereich eine Oberfläche einschließt, die mit okklusalen Zahnoberflächen in Kontakt kommt, und mindestens ein RFID-Tag einen oberen und einen unteren Abschnitt (14a, 14b) umfasst, die sich zusammen bewegen, wenn der Aligner getragen wird.

7. Aligner nach Anspruch 5 oder 6, wobei der verformbare Bereich eine Oberfläche einschließt, die den labialen Zahnoberflächen zugewandt ist, und mindestens ein RFID-Tag einen inneren und einen äußeren Abschnitt umfasst, die sich zusammen bewegen, wenn der Aligner getragen wird.

8. Alignersystem, umfassend:
den Aligner (10) nach einem der Ansprüche 1 bis 7;
eine RFID-Abfrageeinheit (20) zum Abfragen des einen oder der mehreren RFID-Tags und zum Empfangen einer reflektierten Reaktion; und
eine Steuereinheit (28) zum Bestimmen anhand der reflektierten Reaktion, ob der Aligner getragen wird.

9. System nach Anspruch 8, wobei die Steuereinheit (28) angepasst ist, um anhand der empfangenen reflektierten Reaktion ein Niveau der Passung des Aligners zu dem Benutzer zu bestimmen.

10. System nach Anspruch 8 oder 9, wobei die Steuereinheit (28) angepasst ist, um anhand der empfangenen reflektierten Reaktion Zahnpositionsinformationen zu bestimmen.

## Revendications

1. Aligneur orthodontique (10), comprenant :
une structure de support (12) à placer dans la bouche d'un utilisateur, dans lequel la structure de support présente une forme qui s'adapte aux dents de l'utilisateur et présente une région déformable ; et
au moins une étiquette RFID passive (14) montée sur la structure de support, **caractérisé en ce que** l'étiquette RFID passive comprend des première et seconde portions (14a, 14b) couplées à la région déformable, dans lequel les première et seconde portions comprennent chacune une portion d'antenne respective, qui définissent ensemble l'antenne de l'étiquette RFID,
dans lequel une position relative entre les première et seconde portions est réglable par déformation de la région déformable entre au moins une première position relative lorsque l'aligneur orthodontique est porté par l'utilisateur et une seconde position relative lorsque l'aligneur orthodontique n'est pas porté,
dans lequel une réponse de l'étiquette RFID à un signal d'interrogation externe dépend de la position relative entre les première et seconde portions.

2. Aligneur selon la revendication 1, comprenant une pluralité d'étiquettes RFID (14), chacune comprenant des première et seconde portions couplées à la région déformable.

3. Aligneur selon la revendication 2, comprenant une pluralité d'étiquettes RFID (14) associées à une ou plusieurs dents individuelles à surveiller.

4. Aligneur selon l'une quelconque des revendications 1 à 3, dans lequel la position relative entre les première et seconde portions est réglable entre un premier état dans lequel l'étiquette RFID n'est pas fonctionnelle et ne répondra pas à un signal d'interrogation et un second état dans lequel l'étiquette RFID est fonctionnelle et répondra à un signal d'interrogation.

5. Aligneur selon l'une quelconque des revendications 1 à 4, dans lequel la structure de support (12) comprend une protection qui s'adapte aux dents d'une mâchoire.

6. Aligneur selon la revendication 5, dans lequel la région déformable inclut une surface qui entre en contact avec les surfaces occlusales des dents, et au moins une étiquette RFID comprend des portions supérieure et inférieure (14a, 14b) qui se déplacent ensemble lorsque l'aligneur est porté.

7. Aligneur selon la revendication 5 ou 6, dans lequel la région déformable inclut une surface qui fait face aux surfaces labiales des dents, et au moins une étiquette RFID comprend des portions interne et externe qui se déplacent ensemble lorsque l'aligneur est porté.

8. Système d'aligneur, comprenant :
l'aligneur (10) selon l'une quelconque des revendications 1 à 7 ;
une unité d'interrogation RFID (20) pour interroger les une ou plusieurs étiquettes RFID et recevoir une réponse réfléchie ; et
un dispositif de commande (28) pour déterminer si l'aligneur est porté à partir de la réponse réfléchie.

9. Système selon la revendication 8, dans lequel le dispositif de commande (28) est adapté pour déterminer un niveau d'adaptation de l'aligneur à l'utilisateur à partir de la réponse réfléchie reçue.

10. Système selon la revendication 8 ou 9, dans lequel le dispositif de commande (28) est adapté pour déterminer des informations de position de dent à partir de la réponse réfléchie reçue.
